# EUROPEAN PATENT SPECIFICATION

(11) **EP 3 138 480 B1**
(45) Date of publication and mention of the grant of the patent: **26.01.2022**
(21) Application number: 16186904.5
(22) Date of filing: 01.09.2016
(51) Int. Cl.: A61B 5/00, A61M 21/02, G16H 40/00, A61B 5/11, A61B 5/0205, A61B 5/024, A61B 5/08, A61M 21/00

(54) **METHOD AND SYSTEM TO OPTIMIZE LIGHTS AND SOUNDS FOR SLEEP**
VERFAHREN UND SYSTEM ZUR OPTIMIERUNG VON LICHT UND TON ZUM SCHLAFEN
PROCEDE ET SYSTEME D'OPTIMISATION DE LUMIERES ET DE SONS POUR LE SOMMEIL

(30) Priority: 03.09.2015 US 201514844571
(43) Date of publication of application: 08.03.2017
(73) Proprietor: Withings, 92130 Issy-les-Moulineaux (FR)
(72) Inventor: BONVALLET, Eglantine, 75016 Paris (FR); VILLARD, Joffrey, 75014 Paris (FR); HUTCHINGS, Cédric, 92130 Issy les Moulineaux (FR)
(74) Representative: Plasseraud IP

(56) References cited:
- WO-A2-2007/052108
- US-A1- 2006 031 288
- US-A1- 2008 077 020
- US-A1- 2011 230 790
- US-A1- 2014 317 119
- US-A1- 2015 141 852
- US-A1- 2015 164 409
- US-A1- 2015 187 199

## Description

### FIELD OF THE INVENTION

The present invention relates to methods and systems that can help a user to improve sleep. More particularly it concerns light and sound sequences designed to accompany a falling asleep phase of the user, and light and sound sequences designed to accompany a awakening phase of the user.

### BACKGROUND OF THE DISCLOSURE

In the conventional art, it is known, for example from document WO2005066868, to optimise the sleeping general conditions (ambient temperature, air humidity) in order to improve the sleep quality of the user.

Document US 2015/0187199 discloses to monitor the brainwave signal of a user to determine a sound or light program to display.

Besides, it is known to optimise the time to wake up within a user-defined wakeup window for a snooze alarm function, for example from document US2011230790.

However, it has been found by the inventors that the sleep quality can be further improved, especially regarding two important sleep phases, namely the beginning (falling asleep phase) and the end (awakening phase).

### SUMMARY OF THE DISCLOSURE

The invention is set out in the appended set of claims.

The device according to the invention advantageously proposes the more relevant light and sound program(s) to the user, according to the current time, day in the week, according to previous daytime activities performed by the user, and according to general preferences of the user. Thereby, the relaxation phase and falling sleep phase are more appropriate than default or standard light and sound programs. Also, the wake-up sequence can be improved, especially the user mood at wakeup, it favors a 'feel good wakeup'.

### BRIEF DESCRIPTION OF THE DRAWINGS

Other features and advantages of the invention appear from the following detailed description of one of its embodiments, given by way of non-limiting example, and with reference to the accompanying drawings, in which:
- Figure 1 shows a general schematic view of a system in which the disclosed method can be carried out,
- Figure 2 is a block diagram of the system of figure 1,
- Figure 3 shows a time chart illustrating various phases of day and night, especially sleep phases,
- Figure 4 illustrates the steps of the disclosed method carried out at the central server,
- Figure 5 illustrates generally the steps of the disclosed method,
- Figure 6 illustrates a time chart showing sequences of messages exchanged between the user devices and the central server,

### DETAILLED DESCRIPTION OF THE DISCLOSURE

In the figures, the same references denote identical or similar elements.

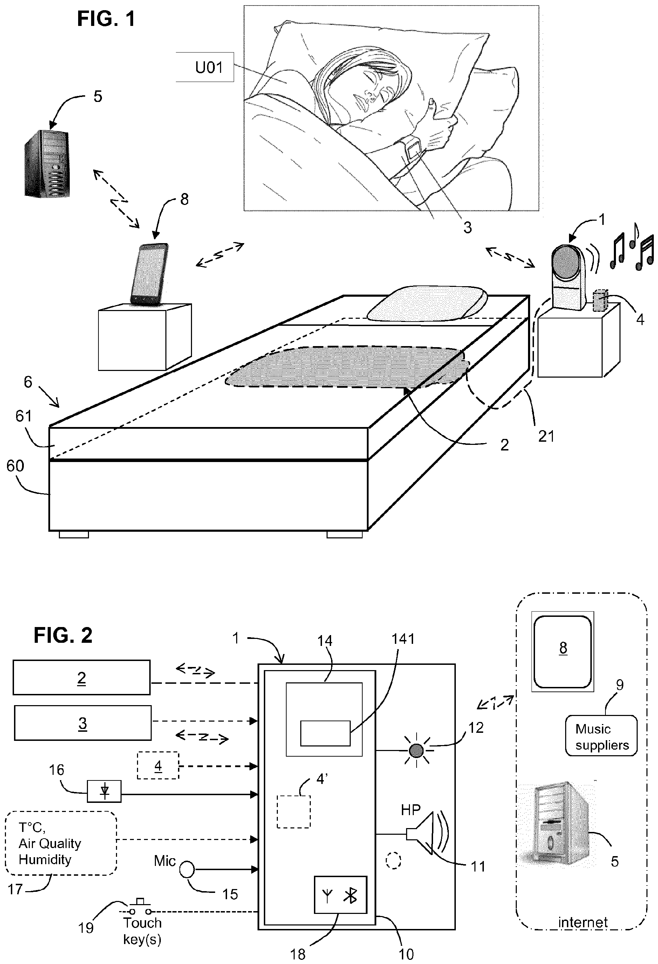

Figure 1 shows a bed or 'bedding' **6,** intended to be used by a user **U01,** named hereafter 'first user'.

The bedding **6** includes a main mattress **60** above which is placed a **detection device 2** having an active sensing portion which will be described below and for which the position advantageously approximately corresponds to one of the pressure points of the body of the person lying on the mattress.

Placed above the detection device 2 is a mattress pad, a mattress topper **61,** a fitted sheet, or other layer on which the individual using the bedding may lie. In another embodiment not shown, the detection device 2 may be installed under the main mattress **60.**

The detection device 2, which is also called hereafter **a sensing mat 2,** can rely on a pneumatic bladder principle or on a piezo-electric mesh principle. More detailed about the detection device can be found in the publication US2015141852 of the same applicant, which is incorporated here by reference.

The sensing mat 2 is configured to detect, by ballistography, at least the movements, heart rate and respiratory rate (by detecting movements or even micro-movements) of an individual lying on the mattress, in order to monitor the sleep of this individual.

Besides, Figures 1-2 shows a **bedside device 1** typically placed on a bed side table; the bedside device 1 is configured to receive signals coming from the sensing mat 2 via a wired link **21** or a wireless connection.

Also, the first user U01 usually wears an **activity tracker 3** which is usually a personal device. In the sown example, the user wears activity tracker 3 at the wrist, but it can also be worn at other locations. The activity tracker can also be formed as a miniaturized ear plug, a finger ring, a necklace, or the like ......

According to another embodiment (not shown) the above mentioned activity tracker 3 and the above mentioned sensing mat 2 can be replaced by a **single bio parameter sensor** which is capable of sensing in real time the heart rate the respiratory rate and possibly other parameters like blood pressure, oxygen concentration in blood, body temperature, etc...

This kind of single bio parameter sensor can be in the form of wristband, an ear plug, a finger ring, a necklace, a chest pad, or the like.

Besides, the system may additionally comprise an **alcohol level measurement device 4.** This device can be an ethylometer with an exhalation port and a alcohol concentration sensor, as known for example from document US4678057.

It is not excluded that the above-mentioned single bio parameter sensor can also measure the user's current alcohol concentration.

Besides, the system comprises a personal portable electronic device, in particular a **Smartphone 8.** This device comprises a known easy-to-use user interface, namely with a display and a touch pad or touch screen. The Smartphone 8 has an internet wireless connection and a short range wireless connection like Bluetooth; it can generally exchange data with the bedside device and Internet servers. The smartphone 8 runs an application known here as 'sleep management application' which enables the user to conveniently use the whole system.

Advantageously, the system comprises at least a **central server 5** connected to the Internet. The central server has a large amount of resources, extensive computing power, large memory hard-disk space and so on. Of course, the above-mentioned central server can also be a set of various resources on the Internet 'cloud'.

The central server is configured to be connected not only to the smartphone 8 and/or the bedside device of the first user **U01,** but also to similar smartphones and/or similar bedside devices of a plurality of other users **Uxx.**

The central server is handling data about users of the systems similar to the one depicted in figure 1 and the central server particularly provides assistance to improve the sleep pf users U01,Uxx.

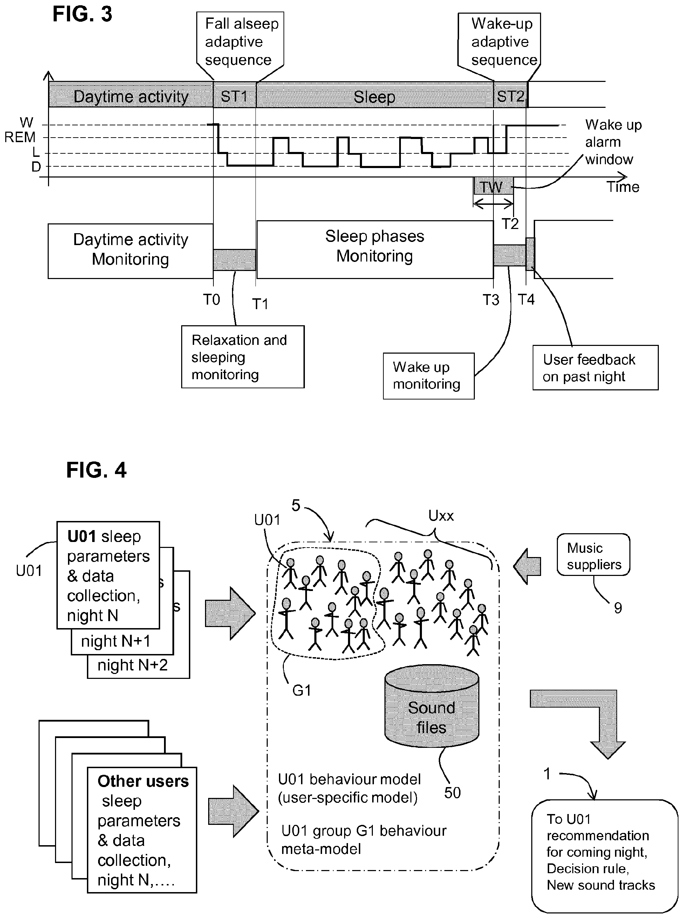

As shown in figure 3, a complete sleep sequence begins by a **fall asleep sequence** denoted **ST1,** and terminates with an **awakening sequence** denoted **ST2.**

According to the present invention, both sequences ST1,ST2 are accompanied by a **light and sound program**, in other words the bedside device is configured to play a **music track** (likewise called generally **"sound file"** hereafter) that can be heard by a user in the bed during these sequences ST1,ST2. Also a light is produced by the bedside device, preferably at least at the same time than the music track.

The music tracks or more generally the sound files can be of any type, song, classics, relaxing music, natural sounds, etc... The music tracks can be downloaded from websites managed by music suppliers **9,** or can be likewise already available in user's digital music player, which content can be transferred to the bedside device.

The bedside device 1 comprises a light source **12,** a loudspeaker **11,** a light sensor **16,** a microphone **15,** a wireless interface **18,** a user interface **19** (for example a touch interface/a movement sensor configured to recognize touches, gestures, and the like), and an electronic board **10** with a control unit **14.**

The bedside device 1 may further comprise one or more of the following components: a temperature sensor, an air quality sensor, a humidity sensor (additional sensors collectively denoted **17**), a display unit (not shown in Fig 2), an integrated alcohol concentration sensor 4'.

The microphone, the light sensor, the temperature sensor and the air quality sensor provided on the bedside device **1** may be of any type known in the art.

The microphone **15** is adapted to detect and record sounds that surround the user, for example noises from inside the room, from the street, from user's breathing, snoring (of either the user, or the user's partner who is sleeping in the same room), etc.

The light sensor **16** is adapted to detect the light that surrounds the user, such as daylight, or artificial light, coming either from the inside (e.g. the house lights), or outside (e.g. street lamps, cars passing by).

The temperature sensor measures the ambient temperature of the room. The air quality sensor may be further divided into carbon dioxide concentration sensor, Volatile Organic Compound (VOC) sensor, other gas or dust sensor, air humidity sensor, and the like; it is adapted to measure and assess the quality of the air in the room.

The microphone **15,** the light sensor **16,** the temperature sensor and the air quality sensor are all connected to the control unit **14.** The control unit **14** is equipped with a processing unit and a memory unit **141,** and is capable of processing the data obtained from the sensors. Preferably, the processing unit is configured to assess the user's sleep cycles based on the data obtained from the sensors.

The light source **12** provided on the bedside device 1 is preferably adapted to emit light of several different colors (e.g. white, red, orange, yellow, green, turquoise, blue, violet; other colors not being excluded). Preferably, the light emitted by the light source may also be of variable intensity. In other words, the light source is able to emit multi-wavelength/multicolor LED lighting programs or static lights. For example, the light source may use a multicolor LED dimming lighting technology.

The loudspeaker **11** may be of any type known in the art. The loudspeaker may be provided directly in the bedside device itself. Alternatively, it may be provided separately, as a bedside-device-driven sound system. The loudspeaker is preferably able to diffuse sounds of frequencies within the audible range.

The light source **12** and the loudspeaker **11** are connected to the control unit **14.** For the falling asleep **ST1** and awakening phases **ST2,** the control unit **14** controls the loudspeaker and/or the light source according to some already mentioned predefined light and sound program.

The bedside device **1** may further be provided with a display unit. The display unit may display various kinds of information, such as time, ambient temperature, level of noise, etc. In the displayed information, the sensory data (such as user's heart rate) obtained from the above-described sensors may be displayed.

The bedside device 1 is provided with a user interface **19.** The user interface **19** may be in the form of physical buttons and/or comprises touch areas (one or more touch screens) and/or movement sensors and/or voice controls. The touch areas and/or movement sensors may be adapted to recognize various predefined or pre-set touches and/or movements, through which the user may communicate with the bedside device **1.** For example, movements may be predefined or pre-set to set the alarm, switch on/off the lights, adjust the light intensity and sound volume, start/stop the light and sound programs (described below), snooze wake program, switch off the bedside device completely, etc. Further, some or all of the touches/gestures may be adjusted by the user to suit him/her best. Also the interface may include a voice recognition function allowing some basic voice controls (Turn On, Turn Off, Next, increase, decrease,...).

In one embodiment, the control unit **14** of the bedside device 1 is configured to analyse the user's sleep cycles, based on the data obtained from the sensing mat 2 and tracking device 3, which provide data about the user's heart rate and variability, breathing frequency and variability, movements etc., and the bedside device 1 assesses the user's sleep cycle, based on this data. Preferably, the bedside device 1 may be configured to recognize a light sleep phase **L,** a deep sleep phase **D**, and an **REM** sleep (also called 'paradoxical' sleep) phase. The bedside device 1 may calculate the sleep quality index **SQI** therefrom; sleep quality index **SQI** is a score which reflects the sufficient duration of sleep phases, their balance, the absence of inadvertent wakeups, etc...

The memory unit **141** may store multiple adaptive sequences, notably in form of light and sound programs, i.e. pre-defined or pre-set lights (colors, intensity, changes in colors and/or intensity of the lights) and sounds (music, relaxing sounds, and the like), which are played under specific circumstances as will be seen further.

The bedside device 1, the sensing mat 2 and the traffic device 3 are configured to help gathering a set of data called **"sleep data"** and at least a part of another set of data called **"sleep context data",** as it will be detailed later.

The smartphone **8** is also involved in gathering some "sleep data" and "sleep context data".

The smartphone **8** is preferably involved in transmitting this information towards the central server **5,** where a direct transmission from the bedside device 1 to central server 5 is not excluded via a routing box or the like.

Generally, the average adult's sleep goes through sleep phases of **REM** sleep (paradoxical sleep) and NREM sleep, namely light sleep **L** and deep sleep **D** (Fig 3).

In average, there are 3 to 5 NREM/REM cycles, with average duration of 90-100 minutes. The NREM sleep is further divided into a light sleep **L** and a deep sleep **D;** in average, the deep sleep phases tend to be longer in 1^{st} part of the night, while the REM sleep tends to be longer in the 2^{nd} part of the night. For example in the light sleep, the user's breath is usually slower than when awake, and the heart rate is slowed down; in deep sleep, the heart rate and breathing frequency is further slowed, the muscles are relaxed and the movements are very little or non-existent; in REM phase, the heart rate and breathing frequency may increase again, and their variability is increased.

The initial phase of sleep, i.e. the falling asleep phase **ST1,** begins at time denoted **T0** and ends at time denoted **T1.**

The final phase of sleep, i.e. the awakening phase **ST2,** begins at time denoted **T3** and ends at time denoted **T4.**

The target wake-up window is defined latest wake-up time denoted **T2,** and a target wakeup window duration **TW** (for example 20 min or 30 min).

### User profile

For each user, it is defined a user profile, comprising at least : gender, age, country of residence. Also, additional information like weight, height, profession, educational background can be included in the user profile. Also, additional information like current general health state, known chronic illness, medicines taken regularly, days worked, working hours, etc can also be included in the user profile.

The user profile is typically entered on the smartphone 'sleep management application', at least once before first use; the user profile can also be completed and updated later.

### Music tracks / sound files

Sound files that are made available at the bedside devices can be obtained from music suppliers **9** like Deezer^{™}, Spotify^{™}, in user's digital music player or the like.

The main storage **50** of sound files is located on the central server **5**, whereas a shortlist of sound files can be stored in the local memory **141** of each bedside device 1.

Each sound file is allocated with complementary parameters such as program description (name, category, tempo/rhythm, artist etc...).

Some program default settings which are made specific to the use in bedside devices like volume, luminosity, color, etc..., can be defined at the central server 5, and associated with each sound file.

### System Functionalities

The system is configured to regularly collect, with regard to the first user U01, **sleep data** and **sleep context data.**

**Sleep context data** and **sleep data** are collected, possibly for each past night, even though it is not a problem that some night(s) are not collected/recorded, such as for example if the users stays one or more night away from home.

The term **"sleep data"** is to be construed broadly in the present specification. The sleep data comprises:
SD1- the light and sound program played for the falling asleep phase ST1,
SD2- the light and sound program played for the awakening phase ST2,
SD3- **bio parameters and ballistographic measurements** and sleep patterns sequence deduced therefrom,
SD4- user feedback as described below,
SD1 and SD2 are also called "program data".

The term **"sleep context data"** encompasses:
SC1- **environmental conditions** such as noise, temperature, humidity,
SC2- geolocation (time zone, sunset & sunrise times), weather conditions,
SC3- previous daytime activity such as physical exercise (sports, playing music, playing games, walking, having sex, ...),
SC4- food intake, alcohol intake, number of coffees taken,
SC5- stress level, fatigue level,
SC6- time to go to sleep.

At the end of the night, the user enters a **feedback** about the night. This user feedback is entered on the smartphone after wakeup, the user feedback being preferably a rating of the past sleep. For example, a qualitative ranking like 'good', 'rather good', 'poor', 'terrible', 'nightmaric', etc. Or a mark between 1 and 10.

Additionally, the user may tick prompted boxes or enter free format comments like : rememberance of dream, ache somewhere, number of conscious wakenings, go-to-toilet, etc.

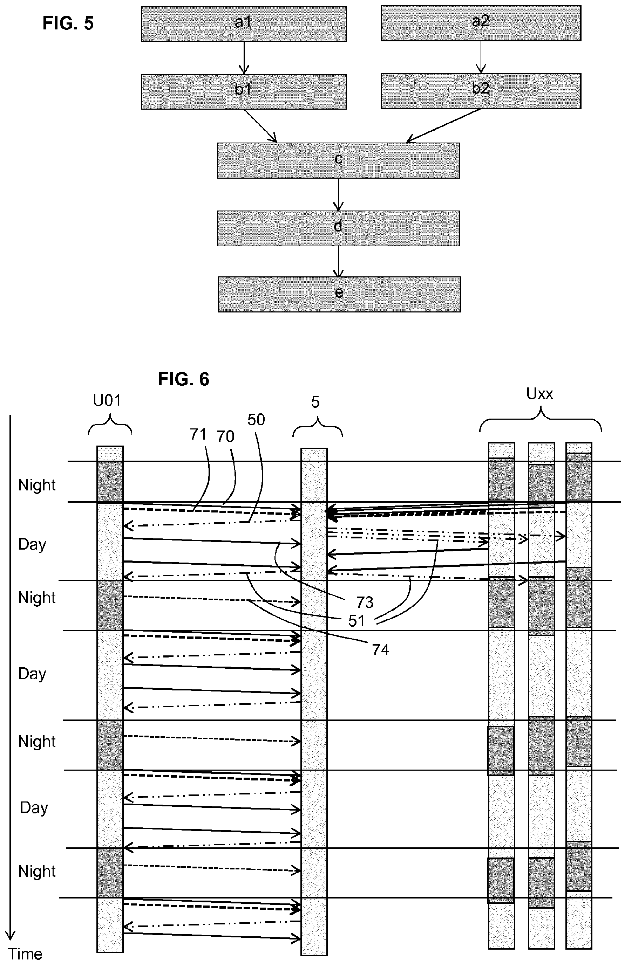

As shown in figure 6, "sleep data" and "sleep context data" are sent to the central server either from time to time, or on a periodical basis, as depicted by arrows **70,71,73,77.** Preferably, "sleep data" **70** is sent at least after termination of each night; possibly the user feedback can be slightly delayed **71,** especially if the user does not enter its feedback, immediately after wakeup.

Sleep context data can be sent to the central server more often during daytime (arrows **74**). Sleep context data can be updated nearly in real time, from the tracking device 3 and/or from the smartphone **8,** without necessarily involving the sensing mat **2** and the bedside device **1.**

**Sleep data** relating to the first user **U01** is sent to the **central server** 5 (Step /a11/) preferably for each 'monitored' night. **Sleep context data** relating to the first user **U01** is sent regularly to the **central server** 5.

The same or similar process of data collection about the sleep as per the first user also occurs for a plurality **of other users Uxx** (Step /a2/). Likewise, sleep context data and sleep data related to the other user are also sent to the **central server** 5 (Step /a21/).

### User-specific model

For each user, from the relevant data collected, the central server 5 builds a **user-specific model** of his/her sleep and sleep background.

Apart from the user profile, **the user-specific model** reflects particularly **sleep behavior** and habits, without excluding factors influencing an individual's sleep for example :
- Average, usual sleep duration
- Average, usual time to go to bed
- Average, usual time to wake up
- Average, usual TV watching duration before going to bed
- usual physical activity level,
- reference values for bio parameters
- list of music programs played falling sleep, with preference ranking (using user's subjective feedback),
- list of music programs played waking up, with preference ranking (using user's subjective feedback),

Also the following factors can be taken into account in the user-specific model:
- Travel habits, notably in different time zones,
- Vacations usual dates,
- days worked in the week,
- specific working hours

The user-specific model also keeps all history data (log) relating to this particular user.

Also variations from one day/night to another about the sleep context data and sleep data are taken into account in the user-specific model.

### Similar users / groups

With all the data gathered at the central server **5,** (also referred to as 'big data'), the server will endeavor to establish **groups of users.**

More precisely, the central server 5 (step /c/) compares various user-specific models to define similar users using behaviour similarities. Practically, the central server 5, and define **groups** of similar users, or set of similar users. The term 'group' is here to be construed broadly; it is not excluded that one user belongs to more than one group; also a new user can be changed from one default group to another one after its user specific model is sufficiently determined.

For instance, a set of users having a user-specific model similar to the one of the first user U01 will be defined as a **first group G1** of users. Let's assume in the following that users U01, U02, U03 belong to group G1.

Each user of a particular **group** has his/her own preferred list of sound files played for phases ST1,ST2.

For instance U01 preferred list of sound files played for phase ST1 is [A1,B1,C1,D1] For instance U01 preferred list of sound files played for phase ST2 is [T1,U1,V1,W1].

For instance U02 preferred list of sound files played for phase ST1 is [A2,B2,C2,D2,A1] For instance U02 preferred list of sound files played for phase ST2 is [T2,U2,V2,W2,Y2].

For instance U03 preferred list of sound files played for phase ST1 is [A3,B3,C3,H3] For instance U03 preferred list of sound files played for phase ST2 is [T3,U3,V3,W3,Z3].

The preferred playlist of sound tracks of the group G1 for phase ST1 will comprise LG1 = [A1,B1,C1,D1,A2,B2,C2,D2 A3,B3,C3,H3]. The preferred playlist of sound tracks of the group G1 for phase ST2 will comprise **LG2** = [T1,U1,V1,W1, T2,U2,V2,W2,Y2, T3,U3,V3,W3,Z3].

### Group meta-model

Other groups are likewise defined. Each group of users is allocated with a **group meta-model** with decision rules and preferred playlist of sound tracks. The preferred playlist of sound tracks of the group can be typically the union of preferred playlist of all users belonging to this group.

A group meta-model typically comprises:
- preferred playlist of sound tracks LG1, LG2
- decision matrix and/or decision analytic rules to order the sound tracks by relevance according to the current time to go to bed, alcohol level, etc

Decision matrix and/or decision analytic rules of the group meta-model is parametrized by the parameters contained in user-specific model such that group meta-model rules are 'applied' to each particular user of this group.

From time to time, or on a periodical basis, the central server 5 sends to all members of one particular group the corresponding updated group meta-model. Practically, for example, the server sends, to the bedside device **1** or to the smartphone 8 of the first user U01, the group meta-model of the first group **G1** (arrows **50,51** in Fig 6). A new transmission of the group meta-model toward the user U01 may be triggered if the sleep context data of U01 has changed significantly.

Assuming that the first user **U01** is going to go to bed, he/she opens the particular corresponding application on his/her smartphone to launch the **"falling asleep sequence"** at the bedside device. The smartphone application prompts a list of recommended music tracks, ordered by relevance induced by the reference meta-model. The first music is considered as a by-default selection, however, the user can select another one in the proposed list. The shortlist proposed to the user is made dependent upon current bio parameters measurements, and also preferably daytime activity summary of the current day.

For example, if **LG1** includes [A1,B1,C1,D1,A2,B2,C2,D2,A3,B3,C3,H3], this list will be ordered by relevance according to the present conditions regarding U01. For instance, if U01 goes to bed early, the ordered proposed shortlist may be [**B1**,C2,C1,D1,A2,B2, A1,A3]; if U01 goes to bed late, the ordered proposed shortlist may be [**A1**,C1,B1,C2, H3,A2,B2, D3,] ; if U01 goes to bed after alcohol intake , the ordered proposed shortlist may be [**D1**,B1,C1, ,C3,H3] ; if U01 goes to bed after jogging he ordered proposed shortlist may be [**C3**,B2,C1, ,C3,A1] (note that in this latter case C3 does not belong to the preferred list of U01).

The proposed order is a result of a complex multi-criteria calculation, taking into account user profile, ranking of each user of the same group, tempo of each sound track, etc.....

Also in the application, it is possible to pre-select an item in a list of recommended music tracks for the **wakeup sequence** in the morning. Here again, the shortlist can be made dependent upon current bio parameters measurements, and also preferably daytime activity summary of the current day. Additional criteria can be taken from sleep quality encountered in the meantime.

For example, if **LG2** includes [T1,U1,V1,W1,T2,U2,V2,W2,Y2, T3,U3,V3,W3,Z3] this list will be ordered by relevance according to the present conditions regarding U01. For instance, if U01 wakeup after a normal night the ordered proposed shortlist may be [**T1**,U1,V1,W1,T2,U2]; if U01 wakeup after a hangover, the ordered proposed shortlist may be [**T2**,T1,T3,W1,V2,U1]; and so on.

Note : the first sound file within the ordered list is intended to be the default selection.

The first user **U01** can also launch the falling asleep sequence directly via the user interface **19** of the bedside device 1.

According to the user-specific model together with group meta-model rules, the most relevant music track is automatically selected for the falling asleep sequence. Preferably, the most relevant music track can be chosen as the first music track of the computed ordered list (as explained above).

Both for falling asleep sequence ST1 and for an awakening sequence ST2, the selected music track is be played together with the attached most relevant light program. The selected music track can be repeated if the sequence is longer than the music track. Otherwise, in a variant that can be selected in user settings, the next music track in the ordered list can be played.

Thanks to the above dispositions, user **U01** benefits from extensive use and experience of numerous other users **Uxx,** particularly numerous **SQI** scores and subjective feedbacks from other users nights.

Note that some sleep context data can come from a connected electronic scale such as for example a 'Smart Body Analyser^{™}' of the present applicant Withings^{™}. Also some sleep context data can come from a connected blood pressure monitor like the 'smart blood pressure monitor' of the present applicant Withings^{™}. Also the activity tracker device 3 can be the product known as 'Pulse'^{™} of the present applicant Withings^{™}.

It is to be noted that the central server manages different time zones in the case the user and the central server are located in different time zones.

## Claims

1. **A method** for optimizing **light and sound programs** for a **falling asleep phase** (ST1) and for an **awakening phase** (ST2) of a **first user** (U01), in a system comprising, for the first user and each of a plurality of **other users,** a **bedside device** (1), at least a **bio parameter sensor** (2,3), a smartphone (8), and additionally for all users at least a **central server** (5), the method comprising the following steps:
**/a1/** collecting, with regard to the **first user** U01, and for each past night, **sleep data** and **sleep context data,** said **sleep data** comprising at least a light and sound program played for the falling asleep phase and for the awakening phase, bio parameters and ballistographic measurements and sleep patterns sequence deduced therefrom, said **sleep context data** comprising at least previous daytime activity and variations from one day/night to another thereof,
/a11/ sending this sleep data and sleep context data to the **central server** (5),
**/a2/** collecting, with regard to a plurality of the **other users,** and for each past night, the same and/or similar sleep data and sleep context data,
/a21/ sending this sleep data and sleep context data to the **central server** (5),
**/b1/** at the central server (5), building, from the data collected, a **user-specific model** of user sleep behavior for the first user,
**/b2/** at the central server (5), building, from the data collected, user-specific models of user sleep behavior for each of the other users,
**/c/** comparing said user-specific models of user sleep behavior
to define **groups** of similar users, including a **first group** of users to which the **first user** belongs to, each group of users being allocated with a **group meta-model** with decision rules and preferred playlist of sound tracks, the group metal model comprising a preferred playlist of soundtracks of the group and the decision rules being parametrized by the parameters contained in user-specific model of sleep behavior to order the sound tracks,
**/d/** sending the group meta-model relative to the first group, from the server to the bedside device or to the smartphone of the first user U01,
**/e/** at the bedside device or at the smartphone, displaying to the first user, using the group meta-model and in function of the **time to go to sleep,** a recommended list of light and sound programs, comprising an ordered list of soundtracks,
or a particular light and sound program, namely a single choice from the recommended list, for the upcoming falling asleep phase and/or the next upcoming **wakeup phase.**

2. The method of claim 1, wherein the sleep data comprises a **user feedback** entered on the smartphone after wakeup, the user feedback being a rating of the past sleep.

3. The method of claim 1, wherein the at least a bio parameter sensor includes at least a **sensing mat** (2) and a personal **activity tracker** (3), the personal activity tracker (3) being in contact with the user's skin.

4. The method of claim 1, in which the system comprises an alcohol level measurement device (4), and wherein at step /e/ current bio parameters measurements include a **current alcohol level.**

5. The method of claim 1, wherein there is provided together with each data on light and sound program, complementary parameters and program default settings.

6. The method of claim 1, wherein the **environmental conditions** are also taken into account in the sleep context data.

7. The method of claim 1, wherein the user-specific model also comprises user general data, user music style preferences, usual physical activity level or current day activity level.

8. The method of claim 1, wherein the meta model also takes into account the **current weather conditions,** the current season, the day in the week or the moon cycle.

9. The method of claim 1, wherein the bio parameters measurements include **heart rate** and **respiratory rate.**

10. The method of claim 1, wherein the method further comprises:
**/f2/** at the bedside device or at the smartphone, displaying to the first user, using the group meta-model and in function of the **duration** of **sleep,** according to a **target wakeup window,** a recommended list of light and sound programs or a particular light and sound program, namely a single choice, for the next upcoming **wakeup phase.**

11. The method of claim 1, wherein the single choice from the recommended list comprises the first soundtrack within the ordered list.

12. The method of claim 1, wherein the recommended list of light and sound programs is ordered by relevance induced by the group meta-model.

13. A **system,** intended to be used by a first user and by each of a plurality of **other users,** the system comprising a plurality of **bedsides devices** (1), a plurality of **sensing mats** (2), a plurality of personal **activity trackers** (3), a plurality of smartphones (8), and additionally at least a **central server** (5), the system being configured to carry out the **method** according to claim 1.

## Patentansprüche

1. Verfahren zur Optimierung von Licht- und Tonprogrammen für eine Einschlafphase (ST1) und für eine Aufwachphase (ST2) eines ersten Benutzers (U01) in einem System, das für den ersten Benutzer und jeden mehrerer anderer Benutzer eine Vorrichtung (1) am Bett, wenigstens einen Bioparametersensor (2, 3), ein Smartphone (8) und zusätzlich für alle Benutzer wenigstens einen zentralen Server (5) aufweist, wobei das Verfahren die folgenden Schritte aufweist:
/a1/ Sammeln von Schlafdaten und Schlafkontextdaten in Bezug auf den ersten Benutzer U01 und für jede vergangene Nacht, wobei diese Schlafdaten wenigstens ein Licht- und Tonprogramm, das für die Einschlafphase und für die Aufwachphase abgespielt wird, Bioparameter und ballistografische Messungen und daraus abgeleitete Schlafmustersequenzen aufweisen, wobei die Schlafkontextdaten wenigstens die Aktivitäten des vorhergehenden Tages und Veränderungen von einem Tag/einer Nacht zum/zur anderen aufweisen,
/a11/ Senden dieser Schlafdaten und Schlafkontextdaten an den zentralen Server (5),
/a2/ Sammeln der gleichen und/oder ähnlicher Schlafdaten und Schlafkontextdaten in Bezug auf mehrere der anderen Benutzer und für jede vergangene Nacht,
/a21/ Senden dieser Schlafdaten und Schlafkontextdaten an den zentralen Server (5),
/b1/ Aufbauen eines benutzerspezifischen Modells für das Benutzerschlafverhalten für den ersten Benutzer aus den gesammelten Daten an dem zentralen Server (5),
/b2/ Aufbauen benutzerspezifischer Modelle des Benutzerschlafverhaltens für jeden der anderen Benutzer an dem zentralen Server (5),
/c/ Vergleichen der benutzerspezifischen Modelle des Benutzerschlafverhaltens, um Gruppen ähnlicher Benutzer zu definieren, welche eine erste Gruppe von Benutzern umfassen, zu denen der erste Benutzer gehört, wobei jeder Benutzergruppe ein Meta-Gruppenmodell mit Entscheidungsregeln und einer bevorzugten Abspielliste von Tonspuren zugeordnet wird, wobei das Meta-Gruppenmodell eine bevorzugte Abspielliste von Tonspuren der Gruppe aufweist und die Entscheidungsregeln durch die in dem benutzerspezifischen Modell des Schlafverhaltens enthaltenen Parameter parametrisiert werden, um die Tonspuren zu ordnen,
/d/ Senden des Meta-Gruppenmodells bezüglich der ersten Gruppe von dem Server an die Vorrichtung am Bett oder an das Smartphone des ersten Benutzers U01,
/e/ dem ersten Benutzer auf der Vorrichtung am Bett oder auf dem Smartphone Anzeigen einer Empfehlungsliste von Licht- und Tonprogrammen, die eine geordnete Liste von Tonspuren aufweisen, unter Verwendung des Meta-Gruppenmodells und als Funktion der Zubettgehzeit, oder eines bestimmten Licht- und Tonprogramms, nämlich einer einzelnen Wahl aus der Empfehlungsliste für die bevorstehende Schlafphase und/oder die nächste bevorstehende Aufwachphase.

2. Verfahren nach Anspruch 1, wobei die Schlafdaten eine Benutzerrückmeldung aufweisen, die nach dem Aufwachen in das Smartphone eingegeben wird, wobei die Benutzerrückmeldung eine Bewertung des vergangenen Schlafs ist.

3. Verfahren nach Anspruch 1, wobei der wenigstens eine Bioparametersensor wenigstens eine Sensormatte (2) und einen persönlichen Aktivitätstracker (3) aufweist, wobei der persönliche Aktivitätstracker (3) in Kontakt mit der Haut des Benutzers ist.

4. Verfahren nach Anspruch 1, wobei das System eine Alkoholpegelmessvorrichtung (4) aufweist, und wobei die aktuellen Bioparametermessungen in Schritt /e/ einen aktuellen Alkoholpegel umfassen.

5. Verfahren nach Anspruch 1, wobei zusammen mit allen Daten über das Licht- und Tonprogramm ergänzende Parameter und Programmvorgabeeinstellungen bereitgestellt werden.

6. Verfahren nach Anspruch 1, wobei die Umgebungsbedingungen in den Schlafkontextdaten ebenfalls berücksichtigt werden.

7. Verfahren nach Anspruch 1, wobei das benutzerspezifische Modell auch allgemeine Benutzerdaten, Musikstilvorlieben des Benutzers und einen normalen physischen Aktivitätspegel oder einen Aktivitätspegel des aktuellen Tages aufweist.

8. Verfahren nach Anspruch 1, wobei das Metamodell auch die aktuellen Wetterbedingungen, die aktuelle Jahreszeit, den Wochentag oder den Mondzyklus berücksichtigt.

9. Verfahren nach Anspruch 1, wobei die Bioparametermessungen die Herzfrequenz und die Atemfrequenz umfassen.

10. Verfahren nach Anspruch 1, wobei das Verfahren ferner aufweist:
/f2/ dem ersten Benutzer auf der Vorrichtung am Bett oder auf dem Smartphone Anzeigen einer Empfehlungsliste von Licht- und Tonprogrammen oder eines bestimmten Licht- und Tonprogramms, nämlich einer einzigen Auswahl für die nächste bevorstehende Aufwachphase, unter Verwendung des Meta-Gruppenmodells und als Funktion der Schlafdauer.

11. Verfahren nach Anspruch 1, wobei die einzige Auswahl aus der Empfehlungsliste die erste Tonspur in der geordneten Liste aufweist.

12. Verfahren nach Anspruch 1, wobei die Empfehlungsliste für Licht- und Tonprogramme nach der durch das Meta-Gruppenmodell induzierten Relevanz geordnet wird.

13. System, das dazu gedacht ist, von einem ersten Benutzer und von jedem mehrerer anderer Benutzer verwendet zu werden, wobei das System mehrere Vorrichtungen (1) am Bett und mehrere Sensormatten (2), mehrere persönliche Aktivitätstracker (3), mehrere Smartphones (8) und zusätzlich wenigstens einen zentralen Server (5) aufweist, wobei das System konfiguriert ist, um das Verfahren nach Anspruch 1 auszuführen.

## Revendications

1. **Procédé** pour optimiser des **programmes de lumières et de sons** pour une **phase d'endormissement** (ST1) et pour une **phase d'éveil** (ST2) d'un **premier utilisateur** (U01), dans un système comprenant, pour le premier utilisateur et chacune d'une pluralité **d'autres utilisateurs,** un **dispositif de chevet** (1), au moins un **capteur de paramètres biologiques** (2, 3), un smartphone (8), et en plus pour tous les utilisateurs au moins un **serveur central** (5), le procédé comprenant les étapes suivantes :
/**a1**/ la collecte, en ce qui concerne le **premier utilisateur** U01, et pour chaque nuit passée, de **données de sommeil** et de **données de contexte de sommeil,** lesdites **données de sommeil** comprenant au moins un programme de lumières et de sons joué pour la phase d'endormissement et pour la phase d'éveil, des paramètres biologiques et des mesures de ballistographie et une séquence de profils de sommeil déduite de ceux-ci, lesdites **données de contexte de sommeil** comprenant au moins une activité de journée précédente et des variations d'un j our/nuit à l'autre de celle-ci,
**/a11/** l'envoi de ces données de sommeil et données de contexte de sommeil au **serveur central** (5),
**/a2/** la collecte, en ce qui concerne une pluralité des **autres utilisateurs,** et pour chaque nuit passée, des données de sommeil et données de contexte de sommeil identiques et/ou similaires,
**/a21/** l'envoi de ces données de sommeil et données de contexte de sommeil au **serveur central** (5),
/**b1**/ au niveau du serveur central (5), la construction, à partir des données collectées,
d'un **modèle spécifique à l'utilisateur** de comportement de sommeil d'utilisateur pour le premier utilisateur,
**/b2/** au niveau du serveur central (5), la construction, à partir des données collectées,
de modèles spécifiques à l'utilisateur de comportement de sommeil d'utilisateur pour les autres utilisateurs,
**/c/** la comparaison desdits modèles spécifiques à l'utilisateur de comportement de sommeil d'utilisateur
pour définir des **groupes** d'utilisateurs similaires, incluant un **premier groupe** d'utilisateurs auquel appartient le **premier utilisateur,** à chaque groupe d'utilisateurs étant alloué un **méta-modèle de groupe** avec des règles de décision et une sélection préférée de pistes sonores, le méta-modèle de groupe comprenant une sélection préférée de pistes sonores du groupe et les règles de décision étant paramétrées par les paramètres contenus dans un modèle spécifique à l'utilisateur de comportement de sommeil pour ordonner les pistes sonores,
**/d/** l'envoi du méta-modèle de groupe relatif au premier groupe, depuis le serveur au dispositif de chevet ou au smartphone du premier utilisateur U01,
**/e/** au niveau du dispositif de chevet ou au niveau du smartphone, l'affichage pour le premier utilisateur, à l'aide du méta-modèle de groupe et en fonction du **temps pour s'endormir,** d'une liste recommandée de programmes de lumières et de sons, comprenant une liste ordonnée de pistes sonores,
ou un programme de lumières et de sons particulier, à savoir un choix unique à partir de la liste recommandée, pour la prochaine phase d'endormissement et/ou la prochaine **phase de réveil** suivante.

2. Procédé selon la revendication 1, dans lequel les données de sommeil comprennent un **retour utilisateur** entré sur le smartphone après réveil, le retour utilisateur étant une évaluation du sommeil passé.

3. Procédé selon la revendication 1, dans lequel l'au moins un capteur de paramètres biologiques inclut au moins un **tapis de détection** (2) et un **suiveur d'activité** personnel (3), le suiveur d'activité personnel (3) étant en contact avec la peau de l'utilisateur.

4. Procédé selon la revendication 1, dans lequel le système comprend un dispositif de mesure de niveau d'alcool (4), et dans lequel à l'étape /e/ des mesures de paramètres biologiques actuels incluent un **niveau d'alcool actuel.**

5. Procédé selon la revendication 1, dans lequel avec chaque donnée concernant un programme de lumières et de sons sont fournis des paramètres complémentaires et des réglages par défaut de programme.

6. Procédé selon la revendication 1, dans lequel les **conditions environnementales** sont également prises en compte dans les données de contexte de sommeil.

7. Procédé selon la revendication 1, dans lequel le modèle spécifique à l'utilisateur comprend également des données générales d'utilisateur, des préférences de style musical d'utilisateur, un niveau d'activité physique habituel ou un niveau d'activité quotidien actuel.

8. Procédé selon la revendication 1, dans lequel le méta-modèle prend également en compte les **conditions météorologiques actuelles,** la saison actuelle, le jour de la semaine ou le cycle de la lune.

9. Procédé selon la revendication 1, dans lequel les mesures de paramètres biologiques incluent la **fréquence cardiaque** et la **fréquence respiratoire.**

10. Procédé selon la revendication 1, dans lequel le procédé comprend en outre :
**/f2/** au niveau du dispositif de chevet ou au niveau du smartphone, l'affichage pour le premier utilisateur, à l'aide du méta-modèle de groupe et en fonction de la **durée** de **sommeil,** en fonction d'une **fenêtre de réveil cible,** d'une liste recommandée de programmes de lumières et de sons ou d'un programme de lumières et de dons particulier, à savoir un choix unique, pour la prochaine **phase de réveil** suivante.

11. Procédé selon la revendication 1, dans lequel le choix unique à partir de la liste recommandée comprend la première piste sonore dans la liste ordonnée.

12. Procédé selon la revendication 1, dans lequel la liste recommandée de programmes de lumières et de sons est ordonnée par pertinence induite par le métal-modèle de groupe.

13. **Système,** destiné à être utilisé par un premier utilisateur et par chaque utilisateur d'une pluralité d'**autres utilisateurs,** le système comprenant une pluralité de **dispositifs de chevet** (1), une pluralité de **tapis de détection** (2), une pluralité de **suiveurs d'activité** personnels (3), une pluralité de smartphones (8), et en plus au moins un **serveur central** (5), le système étant configuré pour réaliser le **procédé** selon la revendication 1.
